# FASCICULE DE BREVET EUROPEEN

(11) **EP 4 259 037 B1**
(45) Date de publication et mention de la délivrance du brevet: **02.12.2026**
(21) Numéro de dépôt: 21851607.8
(22) Date de dépôt: 09.12.2021
(51) Int. Cl.: A61F 2/00, G16H 20/40, G16H 40/63, G16H 50/20

(54) **SYSTÈME IMPLANTABLE COMPRENANT UN RÉSERVOIR À VOLUME VARIABLE**
IMPLANTIERBARES SYSTEM MIT EINEM RESERVOIR MIT VARIABLEM VOLUMEN
IMPLANTABLE SYSTEM COMPRISING A VARIABLE-VOLUME RESERVOIR

(30) Priorité: 10.12.2020 FR 2013004
(43) Date de publication de la demande: 18.10.2023
(73) Titulaire: Uromems, 38320 Eybens (FR)
(72) Inventeur: LAMRAOUI, Hamid, 38410 Vaulnaveys le Haut (FR)
(74) Mandataire: Regimbeau
(86) Numéro de dépôt international: PCT/FR2021/052249
(87) Numéro de publication internationale: WO 2022/123181

(56) Documents cités:
- WO-A1-2016/083428

## Description

### DOMAINE DE L'INVENTION

La présente invention concerne un système implantable dans le corps humain ou animal, comprenant un circuit fluidique avec un réservoir à volume variable.

### ETAT DE LA TECHNIQUE

Des dispositifs médicaux se présentent sous forme de systèmes implantables dans le corps d'un individu humain ou animal. En particulier, de tels dispositifs peuvent correspondre à des sphincters urinaires artificiels utilisés pour lutter contre l'incontinence urinaire, des bandes ou anneaux gastriques adaptées pour restreindre l'estomac en vue de lutter contre l'obésité, des implants péniens gonflables utilisés pour des prothèses érectiles, etc.

De manière connue en soi, le système implantable peut être à fonctionnement hydraulique et peut comprendre notamment un réservoir de fluide à volume variable et un élément gonflable contenant un volume variable de fluide. L'élément gonflable est en liaison fluidique avec le réservoir de fluide à volume variable, de sorte à pouvoir transférer du fluide depuis le réservoir vers l'élément gonflable, et vice-versa.

Dans le cas d'un système implantable occlusif tel qu'un sphincter urinaire artificiel, l'élément gonflable est une manchette occlusive gonflable capable d'occlure sélectivement un conduit anatomique tel qu'un urètre chez l'homme, ou un col vésical chez la femme. Du fluide peut être transféré depuis le réservoir vers la manchette pour augmenter la pression exercée sur le conduit, et inversement depuis la manchette vers le réservoir pour réduire la pression exercée sur le conduit. Ainsi, en fonction du volume de fluide dans la manchette, une pression plus ou moins forte peut être exercée sur le conduit anatomique à occlure.

L'injection et l'aspiration de fluide dans la manchette gonflable nécessaire à l'occlusion du conduit anatomique peuvent être réalisées soit de manière manuelle et passive telle que pour le sphincter urinaire artificiel référencé AMS800 commercialisé par la société American Medical Systems ou l'implant référencé ZSI375 commercialisé par la société Zéphyr, soit de manière automatique et active (à partir d'une source d'énergie électrique par exemple) pour des implants plus évolués.

Le document WO 2016/083428 A1 décrit un tel système occlusif implantable comprenant un réservoir à volume variable en liaison fluidique avec une manchette occlusive.

Le circuit fluidique du système implantable est susceptible de subir une variation temporaire et potentiellement brusque de pression, en particulier une surpression au niveau de l'élément gonflable.

Par exemple, dans le cas de sphincters urinaires artificiels, une activité physique ou un changement de posture de l'individu dans lequel le système est implanté, par exemple lorsque l'individu est assis sur une selle de vélo, est susceptible d'engendrer une surpression dans la manchette gonflable. Il existe également un risque de surpression lors de l'introduction d'un cathéter dans l'urètre de l'individu, l'aiguille du cathéter présentant un certain diamètre et son introduction dans l'urètre entraînant une augmentation du diamètre de l'urètre, donc une augmentation de la pression dans la manchette.

Or, de telles variations de pression dans le circuit fluidique sont à éviter. Dans le cas de sphincters urinaires artificiels, les surpressions sont susceptibles de dégrader les tissus autour desquels la manchette est agencée, par exemple en entraînant des lésions de ces tissus. Il est donc nécessaire de maîtriser rapidement ces surpressions afin de limiter le risque correspondant de dégradation des tissus.

Des systèmes de pompage actuels utilisés dans d'autres domaines proposent d'ajouter une membrane déformable associée à un ressort, par exemple, pour compenser des surpressions dans le système. Néanmoins, ces systèmes de pompage seraient difficiles à inclure dans un système implantable car ils en complexifieraient grandement la structure, et poseraient des problèmes d'étanchéité, de biocompatibilité, de compacité et d'efficacité. En outre, la déformation de la membrane serait susceptible de modifier excessivement le volume du réservoir, ce qui entraînerait une perte de précision quant à la pression imposée dans le circuit fluidique.

### EXPOSE DE L'INVENTION

Un but de l'invention est de proposer un système implantable qui permette de s'affranchir des inconvénients des systèmes existants. En particulier, le système implantable proposé permet de compenser rapidement, en partie ou entièrement, une variation de pression dans le circuit fluidique.

Un autre but de l'invention est de proposer un système implantable biocompatible, étanche, présentant un encombrement minimal et minimisant la consommation d'énergie nécessaire à la compensation de la surpression.

Selon un premier aspect, l'invention concerne un système implantable dans un corps humain ou animal selon la revendication 1 et les revendications dépendantes.

Selon un deuxième aspect, l'invention concerne un ensemble selon la revendication 14 comprenant un système implantable selon le premier aspect et une télécommande adaptée pour être utilisée par un individu dans lequel le système est implanté. Le système implantable et la télécommande comprennent des moyens de communication adaptés pour communiquer entre eux, et la télécommande est adaptée pour commander un déplacement de la deuxième partie par l'actionneur.

### DESCRIPTION DES FIGURES

D'autres caractéristiques, buts et avantages de la présente invention apparaîtront à la lecture de la description détaillée qui va suivre, donnée à titre d'exemple non limitatif, qui sera illustrée par les figures suivantes :
La figure 1 représente une vue schématique d'un système implantable selon un mode de réalisation de l'invention.
La figure 2 représente une vue en coupe schématique d'un système implantable selon un mode de réalisation de l'invention.
Les figures 3a et 3b représentent des vues en coupe schématiques d'un système implantable selon un mode de réalisation de l'invention, respectivement sans et avec déformation de la deuxième partie.

### DESCRIPTION DETAILLEE DE L'INVENTION

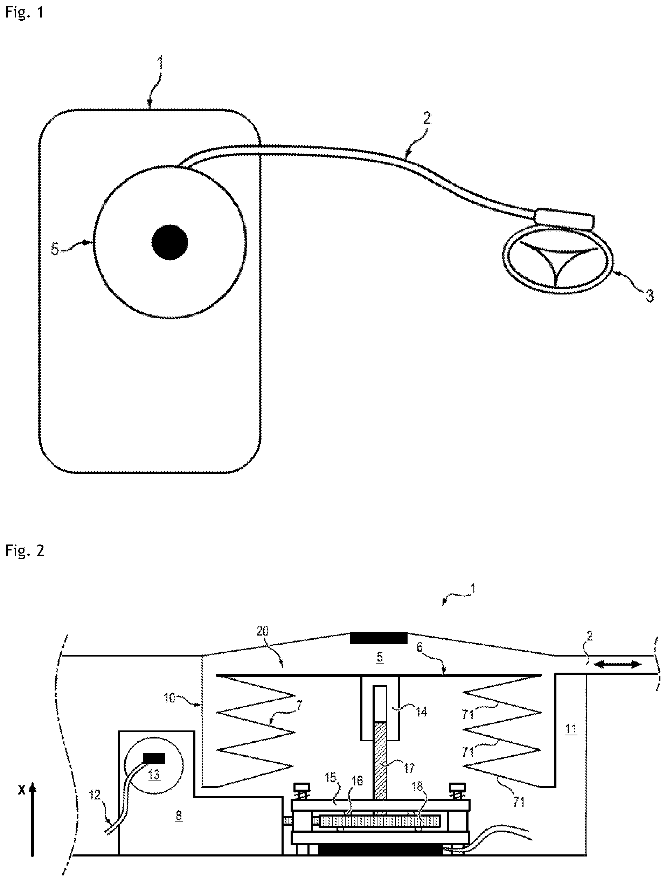

Un système implantable dans un corps humain ou animal est illustré à titre d'exemple non limitatif en figures 1, 2, 3a et 3b.

Le système implantable comprend :
- un circuit fluidique comprenant :
   - un élément gonflable 3 contenant un volume variable d'un fluide ;
   - un réservoir 5 de fluide à volume variable, ledit réservoir 5 comprenant une première partie 10 fixe et une deuxième partie 20 mobile par rapport à la première partie 10 ;
   - une liaison fluidique 2 entre le réservoir 5 et l'élément gonflable 3 ; et
- un actionneur 8 couplé mécaniquement à la deuxième partie 20 pour déplacer sélectivement la deuxième partie 20 par rapport à la première partie 10 de sorte à faire varier un volume du réservoir 5 jusqu'à atteindre un volume déterminé.

La deuxième partie 20 est en outre déformable élastiquement, de sorte que lorsque la deuxième partie 20 est fixe par rapport à la première partie 10, la deuxième partie 20 est adaptée pour être déformée mécaniquement au moins en réponse à une variation de pression dans le circuit fluidique, de sorte à ajuster le volume du réservoir 5 par rapport au volume déterminé pour compenser au moins partiellement ladite variation de pression dans le circuit fluidique.

La deuxième partie 20 du réservoir 5 de fluide du système implantable est ainsi à la fois mobile et déformable élastiquement. La variation de pression dans le circuit fluidique peut être compensée en partie ou entièrement.

Le terme de « pression dans le circuit fluidique » est utilisé pour désigner la pression établie dans chacun des éléments du circuit fluidique, c'est-à-dire dans le réservoir 5, dans l'élément gonflable 3, et dans la liaison fluidique 2.

Le terme de « pression de consigne » est utilisé pour désigner la pression souhaitée dans l'élément gonflable 3, c'est-à-dire dans le circuit fluidique.

La deuxième partie 20 est dite « fixe par rapport à la première partie 10 » en l'absence de sollicitation de la deuxième partie 20 par l'actionneur 8, c'est-à-dire en une position donnée de la deuxième partie 20 par rapport à la première partie 10.

Un déplacement de la deuxième partie 20 par l'actionneur 8, la première partie 10 étant fixe, entraîne une variation du volume du réservoir 5. Ainsi, la deuxième partie 20 peut être déplacée jusqu'à atteindre un volume déterminé de réservoir 5 correspondant à la pression de consigne.

Le réservoir 5 à volume variable est adapté pour être rempli d'un fluide. Une variation du volume du réservoir 5 entraîne une variation de la pression dans le circuit fluidique. Plus particulièrement, une diminution du volume du réservoir 5 entraîne un transfert de fluide depuis le réservoir 5 vers l'élément gonflable 3, et entraîne une augmentation de la pression dans le circuit fluidique. A l'inverse, une augmentation du volume du réservoir 5 entraîne un transfert de fluide depuis l'élément gonflable 3 vers le réservoir 5, et entraîne une diminution de la pression dans le circuit fluidique.

Une déformation de la deuxième partie 20, en une position donnée de la deuxième partie 20, résulte d'une variation de pression dans le réservoir 5, qui peut notamment être représentative d'une variation de pression dans l'élément gonflable 3. La deuxième partie 20 peut notamment être adaptée pour se déformer lorsque la pression dans le réservoir 5 dépasse une valeur de pression limite supérieure à la pression de consigne. La déformation de la deuxième partie 20 permet d'ajuster le volume du réservoir 5 par rapport au volume déterminé, pour compenser la variation de pression dans le circuit fluidique, en particulier pour compenser une surpression dans le circuit fluidique.

La déformation de la deuxième partie 20 est une déformation mécanique. Ainsi, le système élimine le risque d'erreurs de mesure ou de panne d'un système électronique, et ne suppose aucun traitement de données par le biais d'un logiciel particulier. La compensation de la variation de pression est donc réalisée de manière fiable et robuste. En outre, la variation de pression est compensée mécaniquement donc instantanément, sans avoir à être détectée au préalable par des capteurs, et sans faire intervenir une quelconque commande de déplacement de la deuxième partie 20. Le système implantable permet donc une compensation plus rapide d'éventuelles variations de pression dans le circuit fluidique, au moment où ces variations de pression interviennent.

Ainsi, le système implantable est préservé, et ne risque pas d'être endommagé par une éventuelle surpression. La surpression dans l'élément gonflable 3 se répercute en effet au niveau du réservoir 5, est alors absorbée immédiatement par la déformation de la deuxième partie 20, de sorte à rétablir dans le circuit fluidique une pression inférieure ou égale à la pression limite, de préférence correspondant sensiblement à la pression de consigne.

En particulier, dans le cas où le système implantable est un sphincter urinaire artificiel, une surpression temporaire peut survenir dans l'élément gonflable 3, par exemple lors d'une activité physique ou d'un changement de posture de l'individu dans lequel le système est implanté, ou lors de l'introduction d'un cathéter dans l'urètre de l'individu. La surpression est compensée par la déformation de la deuxième partie 20, de sorte que le conduit autour duquel l'élément gonflable 3 est agencé, tel que l'urètre ou le col vésical, n'est pas susceptible d'être dégradé par l'application d'une pression trop importante par l'élément gonflable 3. Le risque de lésions du conduit du fait de la présence du sphincter urinaire artificiel est ainsi limité.

La déformation de la deuxième partie 20 est élastique. Ainsi, une fois la variation de pression disparue, la deuxième partie 20 reprend sa forme initiale, le volume du réservoir 5 correspondant de nouveau au volume déterminé.

### Elément gonflable

L'élément gonflable 3 peut être réalisé en matériau biocompatible, tel que du silicone implantable, du polyuréthane implantable, etc. L'élément gonflable 3 peut être réalisé en élastomère biocompatible, par exemple en silicone biocompatible.

L'élément gonflable 3 peut être une manchette occlusive gonflable, en particulier lorsque le système implantable est un sphincter urinaire artificiel. La manchette occlusive gonflable peut être adaptée pour être agencée autour d'un conduit anatomique afin d'obturer sélectivement ledit conduit anatomique. La manchette occlusive gonflable 3 remplie de fluide peut être adaptée pour entourer totalement ou en partie le conduit à occlure.

En variante, l'élément gonflable 3 peut être un implant pénien gonflable, en particulier lorsque le système implantable est une prothèse érectile.

### Liaison fluidique

La liaison fluidique 2 peut consister en une tubulure disposée entre le réservoir 5 et l'élément gonflable 3. Une première extrémité de la tubulure débouche dans le réservoir 5, et une deuxième extrémité de la tubulure débouche dans l'élément gonflable 3.

La liaison fluidique 2 peut être réalisée en matériau biocompatible, tel que du silicone implantable, du polyuréthane implantable, etc. La liaison fluidique 2 peut être réalisée en élastomère biocompatible, par exemple en silicone biocompatible.

### Réservoir à volume variable

Le système implantable est dans un mode « statique » lorsque la deuxième partie 20 est fixe par rapport à la première partie 10, c'est-à-dire en une position donnée de la deuxième partie 20. Le système implantable est dans le mode statique typiquement lorsque le volume de fluide dans le réservoir 5 est adapté au volume de fluide souhaité dans l'élément gonflable 3. Dans ce mode statique, l'actionneur 8 est inactif.

Le système implantable est dans un mode « dynamique » lorsque la deuxième partie 20 est entraînée en déplacement par l'actionneur 8 par rapport à la première partie 10, typiquement lorsque le volume de fluide dans le réservoir 5 doit être ajusté pour obtenir le volume de fluide souhaité dans l'élément gonflable 3.

Dans le mode statique, c'est-à-dire, lorsque la deuxième partie 20 est fixe par rapport à la première partie 10, la deuxième partie 20 peut être dans une configuration de repos lorsque la pression dans le circuit fluidique est une pression de consigne inférieure ou égale à une pression limite, le volume du réservoir 5 correspondant au volume déterminé, et la deuxième partie 20 peut être dans une configuration déformée lorsque la pression dans le circuit fluidique est supérieure à la pression limite, la déformation de la deuxième partie 20 entraînant une augmentation du volume du réservoir 5 adaptée pour rétablir une pression inférieure ou égale à la pression limite dans le circuit fluidique.

Ainsi, la deuxième partie 20 est dans une configuration de repos, c'est-à-dire ne subit pas de déformation, lorsque la pression dans le circuit fluidique est inférieure ou égale à la pression limite et que le réservoir 5 présente le volume déterminé. La déformation de la deuxième partie 20 étant élastique, la deuxième partie 20 reprend sa forme non déformée lorsque le volume du réservoir 5 est égal au volume déterminé et que la pression dans le circuit fluidique est inférieure ou égale à la pression limite.

La deuxième partie 20 peut comprendre une paroi mobile 6 et un soufflet 7 déformable élastiquement s'étendant entre la paroi mobile 6 et la première partie 10. Le soufflet 7 présente une rigidité moindre que la rigidité de la paroi mobile 6. Ainsi, dans le mode dynamique, le déplacement de la deuxième partie 20 résulte du déplacement de la paroi mobile 6, et la déformation de la deuxième partie 20 résulte de la déformation élastique du soufflet 7.

L'actionneur 8 peut être adapté pour commander un déplacement linéaire de la paroi mobile 6 de la deuxième partie 20, le soufflet 7 de la deuxième partie 20 étant adapté pour s'étendre ou se comprimer en fonction dudit déplacement linéaire de la paroi mobile 6 commandé par l'actionneur 8, de sorte à faire varier un volume du réservoir 5.

La deuxième partie 20 est donc extensible ou compressible axialement de sorte à faire varier le volume du réservoir 5 dans le mode dynamique. Le déplacement de la deuxième partie 20 par l'actionneur 8 entraîne un déplacement linéaire correspondant de la paroi mobile 6 de la deuxième partie 20, et entraîne une extension ou une compression du soufflet 7 dans la direction de déplacement linéaire, en particulier dans la direction de l'axe longitudinal X. Le déplacement de la paroi mobile 6, associé à la compression ou à l'extension du soufflet 7, entraîne une variation du volume du réservoir 5.

La première partie 10 peut comprendre une paroi cylindrique s'étendant de manière sensiblement coaxiale à un axe longitudinal X, et une paroi de fond adaptée pour fermer la paroi cylindrique en l'une de ses extrémités. La paroi cylindrique et la paroi de fond définissent ensemble une paroi externe du réservoir 5.

Le soufflet 7 de la deuxième partie 20 peut former une paroi interne du réservoir 5 s'étendant de manière sensiblement coaxiale à l'axe longitudinal X. Le soufflet 7 peut être raccordé à la paroi cylindrique de la première partie 10 en une première extrémité de soufflet 7, et être raccordé à la paroi mobile 6 en une deuxième extrémité de soufflet 7 opposée à la première extrémité. Ainsi, la paroi cylindrique de la première partie 10 ferme le soufflet 7 en sa première extrémité, et la paroi mobile 6 ferme le soufflet 7 en sa deuxième extrémité.

La paroi mobile 6 de la deuxième partie 20 peut s'étendre perpendiculairement à l'axe longitudinal X. L'actionneur 8 est adapté pour déplacer la paroi mobile 6 en translation suivant l'axe longitudinal X.

La paroi cylindrique de la première partie 10 peut présenter une forme sensiblement de cylindre de révolution autour de l'axe longitudinal X. La paroi mobile 6 peut présenter une forme sensiblement de disque présentant un rayon inférieur à un rayon de la paroi cylindrique de la première partie 10. L'axe de révolution de la paroi cylindrique de la première partie 10 est de préférence aligné avec l'axe longitudinal X et passe par le centre du disque de la paroi mobile 6.

L'actionneur 8 peut être choisi parmi tout système électromécanique permettant de transformer une énergie électrique en un mouvement mécanique avec la puissance requise pour permettre le déplacement à une force et à une vitesse requise de la paroi mobile 6 du réservoir 5 à volume variable. L'actionneur 8 peut notamment être un actionneur 8 piézo-électrique, un actionneur 8 électromagnétique pouvant comprendre un moteur électromagnétique avec ou sans balai couplé ou non à un réducteur, un polymère électro-actif ou un alliage à mémoire de forme.

La paroi mobile 6 peut être déplacée en translation suivant l'axe longitudinal X par l'action d'une vis d'entraînement 17 solidaire de la paroi mobile 6. La vis d'entraînement 17 peut s'étendre sensiblement suivant l'axe longitudinal X. Une position de la vis d'entraînement 17 peut correspondre sensiblement à un centre de la paroi mobile 6.

L'actionneur 8 est adapté pour entraîner la vis d'entraînement 17 en rotation, par exemple par le biais de la rotation d'un pignon. Une rotation de la vis d'entraînement 17 autour de l'axe longitudinal X entraîne un déplacement de la paroi mobile 6 le long de l'axe longitudinal X, le soufflet 7 se comprimant ou s'étendant le long de l'axe longitudinal X en conséquence.

Le soufflet 7 peut comprendre une pluralité de convolutions 71 élastiquement déformables. Dans le mode statique, c'est-à-dire lorsque la deuxième partie 20 est fixe par rapport à la première partie 10, les convolutions 71 sont adaptées pour se déformer mécaniquement de sorte à compenser la variation de pression dans le circuit fluidique.

Ainsi, de préférence, la déformation de la deuxième partie 20 correspond à une déformation des convolutions 71 du soufflet 7. En cas de variation de pression dans le réservoir 5 à volume variable dans le mode statique, en particulier en cas de surpression dans le réservoir 5, la variation de pression est reportée sur les convolutions 71 du soufflet 7, ce qui a pour effet de modifier la forme des convolutions 71.

Le soufflet 7 peut être en forme de soufflet 7 d'accordéon. Les convolutions 71 du soufflet 7 correspondent à des plis de l'accordéon.

Les convolutions 71 peuvent être adaptées pour être disposées de manière adjacente les unes par rapport aux autres dans une direction de déplacement de la paroi mobile 6, c'est-à-dire dans la direction de l'axe longitudinal X.

Les convolutions 71 peuvent être adaptées pour se déformer dans une direction de l'axe longitudinal X et/ou dans une direction radiale par rapport à l'axe longitudinal X.

Chaque convolution 71 peut comporter une première portion 711 et une deuxième portion 712 de convolution 71, raccordées l'une à l'autre au niveau d'une jonction. La deuxième portion 712 d'une première convolution 71 peut être raccordé à la première portion 711 d'une deuxième convolution 71 adjacente au niveau d'une jonction.

Un espace formé entre la première portion 711 et la deuxième portion 712 de la première convolution 71 définit un volume de la première convolution 71 qui est extérieur au réservoir 5. Un espace entre la deuxième portion 712 de la première convolution 71 et la première portion 711 de la deuxième convolution 71 adjacente définit un volume qui fait partie du volume du réservoir 5. Une diminution du volume d'une convolution 71 entraîne une augmentation correspondante du volume du réservoir 5.

La première portion 711 et/ou la deuxième portion 712 d'une convolution 71 peut être en forme sensiblement de segment de droite, ou en variante en forme arrondie ou ondulée, lorsque la deuxième partie 20 est dans la configuration de repos. Une forme arrondie ou ondulée des portions 711, 712 de convolution 71 peut faciliter la déformation de la convolution 71.

Un angle peut être défini entre la première portion 711 et la deuxième portion 712 de convolution 71. En particulier, l'angle peut correspondre à un angle intérieur défini à proximité de la jonction entre la première portion 711 et la deuxième portion 712.

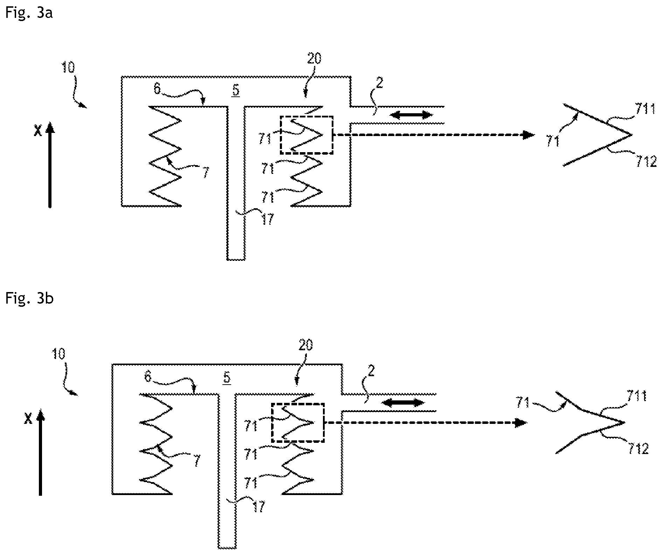

Chaque convolution 71 peut présenter une forme sensiblement de signe supérieur « > » lorsque la deuxième partie 20 est dans une configuration de repos, les deux branches du signe supérieur « > » étant formées respectivement par les deux portions 711, 712 de la convolution 71. La figure 3a illustre un exemple non limitatif dans lequel la deuxième partie 20 est dans la configuration de repos et présente de telles convolutions 71 en forme de signe supérieur « > » formé par des portions 711, 712 en forme de segment de droite. L'angle entre la première portion 711 et la deuxième portion 712 peut correspondre sensiblement à l'angle formé entre les deux branches du signe supérieur « > » défini par les deux portions 711, 712 d'une convolution 71, à proximité d'une jonction entre la première portion 711 et la deuxième portion 712.

Lorsqu'une variation de pression survient dans le réservoir 5, la surpression se répercute sur les deux portions 711, 712 de convolution 71. Chaque portion 711, 712 de la convolution 71 est alors susceptible de se déformer.

En particulier, en cas de surpression dans le circuit fluidique, les convolutions 71 peuvent « s'écraser », les deux portions 711, 712 d'une même convolution 71 se rapprochant, c'est-à-dire le volume de la convolution 71 diminuant. L'angle formé entre les deux portions 711, 712 de la convolution 71 peut être inférieur à l'angle formé entre les deux portions 711, 712 lorsque la deuxième partie 20 est dans une configuration de repos.

Ainsi, dans le cas d'une telle surpression dans le réservoir 5, chaque convolution 71 est déformée de manière à présenter un volume, délimité par un espace entre ses deux portions 711, 712, inférieur au volume de la convolution 71 non déformée. En conséquence, l'espace s'étendant entre la deuxième portion 712 de la première convolution 71 et la première portion 711 de la deuxième convolution 71 adjacente est augmenté par rapport à la configuration de repos. Le volume du réservoir 5 est donc augmenté par rapport au volume déterminé.

La figure 3b illustre un exemple non limitatif dans lequel la deuxième partie 20 est dans une configuration déformée en raison d'une surpression dans le circuit fluidique. Le volume du réservoir 5 est augmenté par rapport au volume du réservoir 5 lorsque la deuxième partie 20 est dans la configuration de repos, ainsi qu'illustré en figure 3a. La déformation des convolutions 71 représentées en figure 3b correspond à une déformation sensiblement selon l'axe longitudinal X des convolutions 71.

Lorsque la deuxième partie 20 est dans la configuration déformée, en particulier lorsqu'une surpression survient dans le réservoir 5, les convolutions 71 peuvent en outre se déformer dans une direction radiale, de sorte à augmenter encore le volume du réservoir 5 et ainsi à compenser la surpression.

Le soufflet 7 peut être adapté pour présenter une rigidité permettant une déformation suffisante pour permettre une compensation d'une éventuelle variation de pression dans le réservoir 5, et une rigidité suffisante pour qu'un déplacement de la deuxième partie 20 corresponde à une modification précise d'un volume du réservoir 5 à volume variable : ainsi, il est possible d'imposer avec précision une quantité de fluide injectée dans l'élément gonflable 3 et un volume déterminé du réservoir 5, tout en permettant une compensation d'une variation de pression temporaire dans le réservoir 5. Plus la rigidité du soufflet 7 diminue, plus le soufflet 7 se déforme pour une variation de pression donnée dans le réservoir 5, et vice-versa.

Le soufflet 7 peut présenter une constante de raideur comprise entre 0,2 et 15 Newton par millimètre (N/mm), de préférence comprise entre 1 et 4 N/mm. La constante de raideur est une caractéristique représentative de la rigidité du soufflet 7. Une telle constante de raideur comprise entre 0,2 et 15 N/mm, de préférence comprise entre 1 et 4 N/mm, correspond à une rigidité du soufflet 7 conforme à la rigidité souhaitée.

La rigidité du soufflet 7, représentée le cas échéant par la constante de raideur du soufflet 7, peut être adaptée notamment à l'aide de l'une ou l'autre ou d'une combinaison des caractéristiques décrites ci-dessous.

Le soufflet 7 peut comprendre entre 3 et 15 convolutions 71 élastiquement déformables, de préférence entre 5 et 10 convolutions 71 élastiquement déformables. Plus le nombre de convolutions 71 du soufflet 7 est important, plus la rigidité du soufflet 7 diminue. Un nombre de convolutions 71 compris entre 3 et 15, de préférence entre 5 et 10, permet d'aboutir à une rigidité du soufflet 7 conforme à la rigidité souhaitée.

Le soufflet 7 peut être réalisé en titane. Le titane permet une déformation adaptée à la compensation, partielle ou totale, d'une variation de pression temporaire dans le réservoir 5.

En outre, le titane est un matériau biocompatible et étanche. Or, le réservoir 5 appartient à un système implantable. En cas de fuite du fluide transmis entre le réservoir 5 et l'élément gonflable 3 ou en cas d'échanges osmotiques, par exemple au niveau de la liaison fluidique 2 ou de l'élément gonflable 3, le fluide est susceptible de passer dans le corps dans lequel le système est implanté. Le titane permet d'éviter que de telles fuites ou échanges osmotiques ne se propage de l'intérieur du boîtier 1 vers l'intérieur du corps et inversement.

Alternativement, le soufflet 7 peut être réalisé en d'autres matériaux, tels que de l'acier inoxydable biocompatible, ou un autre matériau encapsulé dans une couche d'or de sorte à être biocompatible.

Le soufflet 7 peut être soudé aux extrémités et à la base des convolutions 71. Chaque portion 711, 712 d'une convolution 71 peut être soudée en chacune de ses extrémités à une portion 711, 712 adjacente. Ainsi, la première portion 711 d'une première convolution 71 peut être soudée en une première extrémité à la deuxième portion 712 de cette même première convolution 71, et être soudée en une deuxième extrémité opposée à la première extrémité à une deuxième portion 712 d'une deuxième convolution 71 adjacente. Cette caractéristique permet d'améliorer l'étanchéité du soufflet 7.

Le soufflet 7 peut présenter une épaisseur comprise entre 0,01 mm et 0,3 mm, de préférence entre 0,03 mm et 0,15 mm. Une telle épaisseur permet d'aboutir à une rigidité du soufflet 7 conforme à la rigidité souhaitée.

L'homme du métier est à même d'ajuster la géométrie et les dimensions du soufflet en fonction de la pression limite à partir de laquelle il doit se déformer, en s'appuyant le cas échéant sur des simulations numériques et/ou des expérimentations.

### Moyen de mesure de la pression dans le circuit fluidique

Le système implantable peut comprendre un moyen de mesure de la pression dans le circuit fluidique, c'est-à-dire un moyen adapté pour mesurer une pression établie dans le réservoir 5, l'élément gonflable 3, et la liaison fluidique 2.

Le moyen de mesure de la pression dans le circuit fluidique peut être un capteur de pression adapté pour mesurer une pression dans le réservoir 5. En variante, le moyen de mesure de la pression dans le circuit fluidique peut être un capteur de force adapté pour mesurer une force exercée sur la paroi mobile 6 par le fluide contenu dans le réservoir 5. La surface de la paroi mobile 6 étant connue, ladite force exercée sur la paroi mobile 6 est représentative d'une pression dans le réservoir 5.

Dans certaines situations d'utilisation, la pression de consigne définissant la pression souhaitée dans l'élément gonflable 3 peut être choisie de sorte à correspondre à une pression suffisante dans l'élément gonflable 3 pour occlure le conduit naturel sans risquer sa dégradation, en tenant compte éventuellement de l'activité et/ou de la posture du patient. Dans d'autres situations d'utilisation, la pression de consigne peut être choisie suffisamment faible pour libérer le conduit naturel et permettre la miction.

Dans tous les cas, la pression de consigne est inférieure à la pression limite à partir de laquelle la deuxième partie 20 se déforme. La valeur de ladite pression limite peut être fonction de la position de la paroi mobile 6 et / ou de la pression de consigne.

### Unité de commande

Le système implantable peut comprendre une unité de commande.

L'unité de commande peut être configurée pour déterminer un volume de réservoir 5 correspondant à la pression de consigne, et pour commander l'actionneur 8 de sorte à déplacer la deuxième partie 20 du réservoir 5 jusqu'à atteindre le volume déterminé du réservoir 5 correspondant à la pression de consigne.

Plus particulièrement, dans l'exemple illustré en figure 2, l'unité de commande est adaptée pour émettre un ordre de fonctionnement du moteur de l'actionneur 8 électromagnétique dans un sens ou dans l'autre selon qu'une augmentation ou une diminution du volume du réservoir 5 est requise, le système implantable étant alors dans le mode dynamique.

Le système implantable peut passer du mode dynamique au mode statique, la deuxième partie 20 étant en une position donnée, par exemple lorsque l'élément gonflable 3 est gonflé ou lorsque le volume déterminé du réservoir 5 est atteint.

En alternative ou en outre, un déplacement de la deuxième partie 20 peut être commandé par exemple par l'individu dans lequel le système est implanté. Par exemple, dans le cas d'un sphincter urinaire artificiel, lorsque l'individu souhaite uriner, il peut commander un déplacement de la deuxième partie 20 de sorte à augmenter le volume du réservoir 5, ce qui a pour effet d'entraîner un transfert du fluide depuis la manchette occlusive 3 dans le réservoir 5, diminuant ainsi la pression exercée par la manchette 3 sur le conduit à occlure.

L'unité de commande peut, en alternative ou en outre, être adaptée pour commander un déplacement de la deuxième partie 20, à partir d'une pression mesurée par le moyen de mesure de la pression. En particulier, l'unité de commande peut être adaptée pour, lorsque la pression mesurée dans le circuit fluidique ne correspond pas à la pression de consigne, commander un actionnement de l'actionneur 8 adapté pour déplacer la deuxième partie 20 de sorte à modifier le volume du réservoir 5 pour compenser la variation de pression dans le circuit fluidique.

Ainsi, une variation de pression dans le circuit fluidique, telle qu'une surpression, est compensée dans un premier temps par la déformation de la deuxième partie 20, cette compensation étant mécanique, immédiate et directe. La compensation peut être partielle ou totale.

La compensation par la déformation de la deuxième partie 20 est effectuée pendant une durée adaptée pour permettre au moyen de mesure de pression de détecter la variation de pression dans le circuit fluidique et pour permettre à l'unité de commande d'adapter une commande de déplacement de la deuxième partie 20 en conséquence. Ainsi, la variation de pression dans le réservoir 5 est réduite, voire la pression dans le réservoir 5 reste sensiblement constante, pendant la détection et l'adaptation de la commande, du fait de la déformation de la deuxième partie 20.

Dans un deuxième temps, la variation de pression dans le circuit fluidique est compensée par le déplacement de la deuxième partie 20 par rapport à la première partie 10, le système implantable passant en mode dynamique. Ledit déplacement de la deuxième partie 20 est commandé par l'unité de commande par le biais de l'actionneur 8. La deuxième partie 20 peut être déplacée jusqu'à ce que la pression dans le circuit fluidique corresponde à la pression de consigne.

Alors, le déplacement de la deuxième partie 20 par l'actionneur 8 est stoppé, le système implantable passant en mode statique.

En particulier, lorsque la pression mesurée dans le circuit fluidique est supérieure à la pression limite, c'est-à-dire en cas de surpression dans le circuit fluidique, l'unité de commande peut être adaptée pour commander par le biais de l'actionneur 8 un déplacement de la deuxième partie 20 adapté pour augmenter le volume déterminé du réservoir 5, en vue de rétablir dans le circuit fluidique une pression correspondant à la pression de consigne, qui est inférieure ou égale à la pression limite.

### Boîtier

Le réservoir 5 à volume variable et l'actionneur 8 peuvent être intégrés dans un boîtier 1 étanche et biocompatible destiné à être implanté dans le corps de l'individu. L'unité de commande et/ou le moyen de mesure de la pression dans le circuit fluidique peuvent également être intégrés dans le boîtier 1.

Le réservoir 5 à volume variable peut plus particulièrement être délimité par le soufflet 7 en regard de la première partie 10, la paroi mobile 6 et la première partie 10. Le réservoir 5 comprend en outre un orifice permettant de transférer le fluide de et à l'extérieur du réservoir 5 vers la manchette 3 via la liaison fluidique 2.

Le boîtier 1, en particulier le volume intérieur 11 du boîtier 1 entourant le réservoir 5, contient un gaz, par exemple un gaz inerte.

Le boîtier 1 peut être réalisé en titane. Le titane étant un matériau étanche et biocompatible, son utilisation permet de protéger les éléments agencés dans le boîtier 1, en particulier les composants électroniques tel que l'unité de commande, les éventuels capteurs, etc., de l'environnement extérieur.

Le boîtier 1 est étanche pour éviter tout transfert de fluide ou de gaz de ou vers le milieu intracorporel.

Le boîtier 1 est réalisé en un matériau biocompatible et peut par exemple être réalisé en titane implantable et scellé par soudure laser. Un contrôle de l'étanchéité peut notamment être réalisé à l'hélium, pour s'assurer de l'étanchéité totale du boîtier 1 pendant la période pour laquelle le dispositif est implanté.

La figure 2 illustre un exemple d'un système implantable comprenant un réservoir 5 à volume variable et un actionneur 8 électromagnétique intégrés dans un boîtier 1.

L'actionneur 8 comprend un moteur 13 couplé à un réducteur. Un connecteur 12 permet d'alimenter le moteur 13 en fonction d'un ordre de fonctionnement du moteur.

Le réducteur est couplé à une roue dentée 18 qui est elle-même couplée à la vis d'entraînement 17, de sorte à transmettre le couple et la rotation de l'axe du moteur 13 à la vis d'entraînement 17.

Le système d'entraînement comprend en outre une noix 14 couplée à la vis d'entraînement 17 et mobile en rotation sur une butée à billes à double effet autour de l'axe de la vis 17 sous l'effet d'une action d'entraînement par l'actionneur 8. La paroi mobile 6 est couplée à la vis d'entraînement 17 par l'intermédiaire de la noix 14, la noix 14 étant solidaire de la paroi mobile 6 et présentant un taraudage coopérant avec le filetage de la vis 17.

Ainsi, une rotation de la noix 14 entraîne la vis d'entraînement 17 uniquement en translation dans la direction de déplacement de la paroi mobile 6, ce qui a pour effet de déplacer la paroi mobile 6 en translation dans une direction parallèle à l'axe de la vis 17, c'est-à-dire dans la direction de l'axe longitudinal X. Le sens de déplacement de la paroi mobile 6 dépend du sens de rotation du moteur 13.

La roue dentée 18 est logée dans un bloc 15 par l'intermédiaire de roulements à billes 16 qui permettent sa rotation dans le bloc 15.

### Ensemble

Le système implantable décrit ci-dessus, dont un exemple est illustré en figure 1, peut être configuré pour être implanté dans un corps humain ou animal pour obturer sélectivement un conduit anatomique dudit corps humain ou animal pris parmi au moins l'un des conduits suivants : un urètre, un conduit gastrique, un colon ou un rectum.

Un ensemble peut comprendre un système implantable tel que décrit ci-dessus, et une télécommande adaptée pour être utilisée par un individu, par exemple par un individu dans lequel le système est implanté.

Le système implantable et la télécommande comprennent des moyens de communication adaptés pour communiquer entre eux. La télécommande est adaptée pour commander un déplacement de la deuxième partie 20 par l'actionneur 8, en particulier sur la base d'une commande transmise par les moyens de communication de la télécommande aux moyens de communication du système implantable.

Les moyens de communication du système implantable peuvent être intégrés dans le boîtier 1.

D'autres modes de réalisation peuvent être envisagés et une personne du métier peut facilement modifier les modes ou exemples de réalisation exposés ci-dessus ou en envisager d'autres tout en restant dans la portée de l'invention.

## Revendications

1. Système implantable dans un corps humain ou animal, comprenant :
- un circuit fluidique comprenant :
- un élément gonflable (3) contenant un volume variable d'un fluide ;
- un réservoir (5) de fluide à volume variable, ledit réservoir (5) comprenant une première partie (10) fixe et une deuxième partie (20) mobile par rapport à la première partie (10) ;
- une liaison fluidique (2) entre le réservoir (5) et l'élément gonflable (3) ; et
- un actionneur (8) couplé mécaniquement à la deuxième partie (20) pour déplacer sélectivement la deuxième partie (20) par rapport à la première partie (10) de sorte à faire varier un volume du réservoir (5) jusqu'à atteindre un volume déterminé ;
dans lequel la deuxième partie (20) est en outre déformable élastiquement, de sorte que lorsque la deuxième partie (20) est placée dans une position donnée par rapport à la première partie (10), la deuxième partie (20) est adaptée pour être déformée mécaniquement au moins en réponse à une variation de pression dans le circuit fluidique, de sorte à ajuster le volume du réservoir (5) par rapport au volume déterminé pour compenser au moins partiellement ladite variation de pression dans le circuit fluidique ;
le système implantable étant **caractérisé en ce que** la deuxième partie (20) comprend une paroi mobile (6) et un soufflet (7) déformable élastiquement s'étendant entre la paroi mobile (6) et la première partie (10), le soufflet (7) étant adapté pour présenter une rigidité permettant une déformation suffisante pour permettre une compensation d'une éventuelle variation de pression dans le réservoir (5), et une rigidité suffisante pour qu'un déplacement de la deuxième partie (20) corresponde à une modification prédéterminée d'un volume du réservoir (5) ;
et **en ce que** le soufflet (7) comprend une pluralité de convolutions (71) élastiquement déformables, de sorte que, lorsque la deuxième partie (20) est placée dans la position donnée par rapport à la première partie (10), les convolutions (71) sont adaptées pour se déformer mécaniquement afin de compenser la variation de pression dans le circuit fluidique.

2. Système implantable selon la revendication 1, dans lequel lorsque la deuxième partie (20) est fixe par rapport à la première partie (10), la deuxième partie (20) est dans une configuration de repos lorsque la pression dans le circuit fluidique est inférieure ou égale à une pression limite, le volume du réservoir (5) correspondant au volume déterminé, et la deuxième partie (20) est dans une configuration déformée lorsque la pression dans le circuit fluidique est supérieure à la pression limite, la déformation de la deuxième partie (20) entraînant une augmentation du volume du réservoir (5) adaptée pour rétablir la pression limite dans le circuit fluidique.

3. Système implantable selon l'une des revendications 1 ou 2, comprenant en outre un moyen de mesure de pression dans le circuit fluidique et une unité de commande, l'unité de commande étant adaptée pour, lorsque la pression mesurée dans le circuit fluidique ne correspond pas à une pression de consigne, commander un actionnement de l'actionneur (8) adapté pour déplacer la deuxième partie (20) de sorte à modifier le volume du réservoir (5) pour compenser la variation de pression dans le circuit fluidique.

4. Système implantable selon l'une des revendications 1 à 3, dans lequel l'élément gonflable (3) est une manchette occlusive gonflable.

5. Système implantable selon la revendication 4, dans lequel la manchette occlusive gonflable est adaptée pour être agencée autour d'un conduit anatomique afin d'obturer sélectivement ledit conduit anatomique.

6. Système implantable selon l'une quelconque des revendications 1 à 5, dans lequel l'actionneur (8) est adapté pour commander un déplacement linéaire de la paroi mobile (6) de la deuxième partie (20), le soufflet (7) de la deuxième partie (20) étant adapté pour s'étendre ou se comprimer en fonction dudit déplacement linéaire de la paroi mobile (6) commandé par l'actionneur (8) de sorte à faire varier un volume du réservoir (5).

7. Système implantable selon l'une quelconque des revendications 1 à 6, dans lequel la première partie (10) forme une paroi externe du réservoir (5) s'étendant de manière sensiblement coaxiale à un axe longitudinal (X), dans lequel le soufflet (7) de la deuxième partie (20) forme une paroi interne du réservoir (5) s'étendant de manière sensiblement coaxiale à l'axe longitudinal (X), et dans lequel l'actionneur (8) est adapté pour déplacer la paroi mobile (6) en translation suivant l'axe longitudinal (X).

8. Système implantable selon la revendication 7, dans lequel les convolutions (71) sont adaptées pour se déformer dans une direction de l'axe longitudinal (X) et/ou dans une direction radiale par rapport à l'axe longitudinal (X).

9. Système implantable selon l'une quelconque des revendications 1 à 8, dans lequel le soufflet (7) comprend entre 3 et 15 convolutions (71) élastiquement déformables, de préférence entre 5 et 10 convolutions (71) élastiquement déformables.

10. Système implantable selon l'une des revendications 1 à 9, dans lequel le soufflet (7) est réalisé en titane.

11. Système implantable selon l'une des revendications 1 à 10, dans lequel le soufflet (7) présente une épaisseur comprise entre 0,01 mm et 0,3 mm, de préférence entre 0,03 mm et 0,15 mm.

12. Système implantable selon l'une des revendications 1 à 11, dans lequel le soufflet (7) présente une constante de raideur comprise entre 0,2 et 15N/mm, de préférence comprise entre 1 et 4 N/mm.

13. Système implantable selon l'une des revendications 1 à 12, configuré pour être implanté dans un corps humain ou animal pour obturer sélectivement un conduit anatomique dudit corps humain ou animal pris parmi au moins l'un des conduits suivants : un urètre, un conduit gastrique, un colon ou un rectum.

14. Ensemble comprenant un système implantable selon l'une des revendications 1 à 13 et une télécommande adaptée pour être utilisée par un individu dans lequel le système est implanté, dans lequel le système implantable et la télécommande comprennent des moyens de communication adaptés pour communiquer entre eux, dans lequel la télécommande est adaptée pour commander un déplacement de la deuxième partie (20) par l'actionneur (8).

## Patentansprüche

1. In einen menschlichen oder tierischen Körper implantierbares System, umfassend:
- einen Fluidkreis, umfassend:
- ein aufblasbares Element (3), das ein veränderliches Volumen eines Fluids enthält;
- ein Fluidreservoir (5) mit veränderlichem Volumen, wobei das Reservoir (5) einen ersten, feststehenden Teil (10) und einen zweiten, in Bezug auf den ersten Teil (10) beweglichen Teil (20) umfasst;
- eine Fluidverbindung (2) zwischen dem Reservoir (5) und dem aufblasbaren Element (3); und
- einen Aktuator (8), der mechanisch mit dem zweiten Teil (20) gekoppelt ist, um den zweiten Teil (20) in Bezug auf den ersten Teil (10) selektiv zu bewegen, so dass ein Volumen des Reservoirs (5) geändert wird, bis ein bestimmtes Volumen erreicht ist;
wobei der zweite Teil (20) ferner elastisch verformbar ist, so dass, wenn der zweite Teil (20) in Bezug auf den ersten Teil (10) in einer gegebenen Position platziert ist, der zweite Teil (20) ausgelegt ist, um mechanisch zumindest in Reaktion auf eine Druckänderung in dem Fluidkreis verformt zu werden, so dass das Volumen des Reservoirs (5) in Bezug auf das bestimmte Volumen angepasst wird, um die Druckänderung in dem Fluidkreis zumindest teilweise auszugleichen;
wobei das implantierbare System **dadurch gekennzeichnet ist, dass** der zweite Teil (20) eine bewegliche Wand (6) und einen elastisch verformbaren Balg (7) umfasst, der sich zwischen der beweglichen Wand (6) und dem ersten Teil (10) erstreckt, wobei der Balg (7) ausgelegt ist, um eine Steifigkeit aufzuweisen, die eine Verformung erlaubt, die ausreichend ist, um einen Ausgleich einer eventuellen Druckänderung in dem Reservoir (5) zu erlauben, und eine Steifigkeit, die ausreichend ist, damit eine Bewegung des zweiten Teils (20) einer vorbestimmten Änderung eines Volumens des Reservoirs (5) entspricht;
und dass der Balg (7) eine Vielzahl von elastisch verformbaren Falten (71) umfasst, so dass, wenn der zweite Teil (20) in Bezug auf den ersten Teil (10) in der gegebenen Position platziert ist, die Falten (71) ausgelegt sind, um sich mechanisch zu verformen, so dass die Druckänderung in dem Fluidkreis ausgeglichen wird.

2. Implantierbares System nach Anspruch 1, wobei, wenn der zweite Teil (20) in Bezug auf den ersten Teil (10) feststeht, der zweite Teil (20) in einer Ruhekonfiguration ist, wenn der Druck in dem Fluidkreis kleiner oder gleich einem Grenzdruck ist, wobei das Volumen des Reservoirs (5) dem bestimmten Volumen entspricht, und der zweite Teil (20) in einer verformten Konfiguration ist, wenn der Druck in dem Fluidkreis größer als der Grenzdruck ist, wobei die Verformung des zweiten Teils (20) zu einer Vergrößerung des Volumens des Reservoirs (5) führt, die ausgelegt ist, um den Grenzdruck im Fluidkreis wiederherzustellen.

3. Implantierbares System nach einem der Ansprüche 1 oder 2, ferner umfassend ein Mittel zur Druckmessung in dem Fluidkreis und eine Steuereinheit, wobei die Steuereinheit ausgelegt ist, um, wenn der gemessene Druck in dem Fluidkreis nicht einem Solldruck entspricht, eine Betätigung des Aktuators (8) zu steuern, die ausgelegt ist, um den zweiten Teil (20) zu bewegen, so dass das Volumen des Reservoirs (5) geändert wird, um die Druckänderung in dem Fluidkreis auszugleichen.

4. Implantierbares System nach einem der Ansprüche 1 bis 3, wobei das aufblasbare Element (3) eine aufblasbare okklusive Manschette ist.

5. Implantierbares System nach Anspruch 4, wobei die aufblasbare okklusive Manschette ausgelegt ist, um um ein anatomisches Gefäß herum angeordnet zu werden, um das anatomische Gefäß selektiv zu verschließen.

6. Implantierbares System nach einem der Ansprüche 1 bis 5, wobei der Aktuator (8) ausgelegt ist, um eine lineare Bewegung der beweglichen Wand (6) des zweiten Teils (20) zu steuern, wobei der Balg (7) des zweiten Teils (20) ausgelegt ist, um sich in Abhängigkeit von der durch den Aktuator (8) gesteuerten linearen Bewegung der beweglichen Wand (6) auszudehnen oder komprimiert zu werden, so dass ein Volumen des Reservoirs (5) geändert wird.

7. Implantierbares System nach einem der Ansprüche 1 bis 6, wobei der erste Teil (10) eine Außenwand des Reservoirs (5) bildet, die sich im Wesentlichen koaxial zu einer Längsachse (X) erstreckt, wobei der Balg (7) des zweiten Teils (20) eine Innenwand des Reservoirs (5) bildet, die sich im Wesentlichen koaxial zu der Längsachse (X) erstreckt, und wobei der Aktuator (8) ausgelegt ist, um die bewegliche Wand (6) translatorisch gemäß der Längsachse (X) zu bewegen.

8. Implantierbares System nach Anspruch 7, wobei die Falten (71) ausgelegt sind, um sich in einer Richtung der Längsachse (X) und/oder in einer radialen Richtung in Bezug auf die Längsachse (X) zu verformen.

9. Implantierbares System nach einem der Ansprüche 1 bis 8, wobei der Balg (7) zwischen 3 und 15 elastisch verformbare Falten (71), vorzugsweise zwischen 5 und 10 elastisch verformbare Falten (71) umfasst.

10. Implantierbares System nach einem der Ansprüche 1 bis 9, wobei der Balg (7) aus Titan hergestellt ist.

11. Implantierbares System nach einem der Ansprüche 1 bis 10, wobei der Balg (7) eine Dicke zwischen 0,01 mm und 0,3 mm, vorzugsweise zwischen 0,03 mm und 0,15 mm, aufweist.

12. Implantierbares System nach einem der Ansprüche 1 bis 11, wobei der Balg (7) eine Steifigkeitskonstante zwischen 0,2 und 15 N/mm, vorzugsweise zwischen 1 und 4 N/mm, aufweist.

13. Implantierbares System nach einem der Ansprüche 1 bis 12, das konfiguriert ist, um in einen menschlichen oder tierischen Körper implantiert zu werden, um ein anatomisches Gefäß des menschlichen oder tierischen Körpers selektiv zu verschließen, das aus mindestens einem der folgenden Gefäße ausgewählt ist: einer Harnröhre, einem Magenkanal, einem Kolon oder einem Rektum.

14. Anordnung, umfassend ein implantierbares System nach einem der Ansprüche 1 bis 13 und eine Fernbedienung, die zur Verwendung durch ein Individuum ausgelegt ist, in welches das System implantiert ist, wobei das implantierbare System und die Fernbedienung Kommunikationsmittel umfassen, die ausgelegt sind, um miteinander zu kommunizieren, wobei die Fernbedienung ausgelegt ist, um eine Bewegung des zweiten Teils (20) durch den Aktuator (8) zu steuern.

## Claims

1. A system that is implantable in a human or animal body, comprising:
- a fluid circuit comprising:
- an inflatable element (3) containing a variable volume of a fluid;
- a variable-volume fluid reservoir (5), said reservoir (5) comprising a stationary first part (10) and a second part (20) movable relative to the first part (10);
- a fluidic connection (2) between the reservoir (5) and the inflatable element (3); and
- an actuator (8), mechanically coupled to the second part (20) in order to selectively move the second part (20) relative to the first part (10) so as to vary a volume of the reservoir (5) until a determined volume is attained;
wherein the second part (20) is also elastically deformable, such that, when the second part (20) is placed in a given position relative to the first part (10), the second part (20) is adapted for being mechanically deformed at least in response to a variation in pressure in the fluid circuit, so as to adjust the volume of the reservoir (5) relative to the determined volume in order to compensate, at least in part, said variation in pressure in the fluid circuit;
the implantable system being **characterized in that** the second part (20) comprises a movable wall (6) and an elastically deformable bellows (7) extending between the movable wall (6) and the first part (10), the bellows (7) being adapted to have a stiffness allowing a sufficient deformation to permit compensation of a possible variation in pressure in the reservoir (5), and a stiffness sufficient for a movement of the second part (20) to correspond to a predetermined change in a volume of the reservoir (5);
and **in that** the bellows (7) comprises a plurality of elastically deformable convolutions (71), such that, when the second part (20) is placed in the given position relative to the first part (10), the convolutions (71) are adapted for mechanically deforming so as to compensate the variation in pressure in the fluid circuit.

2. The implantable system according to claim 1, wherein, when the second part (20) is stationary relative to the first part (10), the second part (20) is in a rest configuration when the pressure in the fluid circuit is less than or equal to a limit pressure, the volume of the reservoir (5) corresponding to the determined volume, and the second part (20) is in a deformed configuration when the pressure in the fluid circuit is greater than the limit pressure, the deformation of the second part (20) causing an increase in the volume of the reservoir (5) adapted for re-establishing the limit pressure in the fluid circuit.

3. The implantable system according to one of claims 1 or 2, further comprising a means for measuring pressure in the fluid circuit and a control unit, the control unit being adapted, when the pressure measured in the fluid circuit does not correspond to a setpoint pressure, to control an actuation of the actuator (8) adapted for moving the second part (20) so as to modify the volume of the reservoir (5) in order to compensate the variation in pressure in the fluid circuit.

4. The implantable system according to one of claims 1 to 3, wherein the inflatable element (3) is an inflatable occlusive cuff.

5. The implantable system according to claim 4, wherein the inflatable occlusive cuff is adapted for being arranged around an anatomical conduit in order to selectively close said anatomical conduit.

6. The implantable system according to any one of claims 1 to 5, wherein the actuator (8) is adapted for controlling a linear movement of the movable wall (6) of the second part (20), the bellows (7) of the second part (20) being adapted for extending or compressing according to said linear movement of the movable wall (6) controlled by the actuator (8) so as to vary a volume of the reservoir (5).

7. The implantable system according to any one of claims 1 to 6, wherein the first part (10) forms an outer wall of the reservoir (5) extending substantially coaxially with a longitudinal axis (X), wherein the bellows (7) of the second part (20) form an inner wall of the reservoir (5) extending substantially coaxially with the longitudinal axis (X), and wherein the actuator (8) is adapted for moving the movable wall (6) in translation along the longitudinal axis (X).

8. The implantable system according to claim 7, wherein the convolutions (71) are adapted for deforming in a direction of the longitudinal axis (X) and/or in a radial direction with respect to the longitudinal axis (X).

9. The implantable system according to any one of claims 1 to 8, wherein the bellows (7) comprises between 3 and 15 elastically deformable convolutions (71), preferably between 5 and 10 elastically deformable convolutions (71).

10. The implantable system according to one of claims 1 to 9, wherein the bellows (7) is made of titanium.

11. The implantable system according to one of claims 1 to 10, wherein the bellows (7) has a thickness between 0.01 mm and 0.3 mm, preferably between 0.03 mm and 0.15 mm.

12. The implantable system according to one of claims 1 to 11, wherein the bellows (7) has a stiffness constant between 0.2 and 15 N/mm, preferably between 1 and 4 N/mm.

13. The implantable system according to any one of claims 1 to 12, designed to be implanted in a human or animal body in order to selectively close an anatomical conduit of said human or animal body, taken from at least the following conduits: a urethra, a gastric conduit, a colon or a rectum.

14. An assembly comprising an implantable system according to any one of claims 1 to 13 and a remote control adapted for being used by an individual in whom the system is implanted, wherein the implantable system and the remote control comprise communication means adapted for communicating between them, wherein the remote-control is adapted for controlling a movement of the second part (20) by the actuator (8).
